(19)

Europäisches
Patentamt
European
Patent Office
Office européen
des brevets

(11)     **EP 2 660 316 A1**

(12)     **EUROPEAN PATENT APPLICATION**

(43) Date of publication:
**06.11.2013 Bulletin 2013/45**

(51) Int Cl.:
*C12N 5/0735* (2010.01)      *C12N 7/00* (2006.01)

(21) Application number: **12166489.0**

(22) Date of filing: **02.05.2012**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(83) **Declaration under Rule 32(1) EPC (expert
solution)**

(71) Applicants:
• **Helmholtz-Zentrum für Infektionsforschung
GmbH
38124 Braunschweig (DE)**
• **Friedrich-Loeffler-Institut
17493 Greifswald (DE)**
• **National Key Laboratory of Biochemical
Engineering
Zhangguancun, Peking (CN)**

(72) Inventors:
• **Wirth, Manfred
38124 Braunschweig (DE)**
• **Sun, Lijing
100190 Peking (CN)**
• **Su, Zhiguo
100192 Beijing (CN)**
• **Riebe, Roland
18519 Sundhagen (DE)**
• **Jäger, Volker
38104 Braunschweig (DE)**
• **Konisch, Nadine
31234 Edemissen (DE)**

(74) Representative: **Taruttis, Stefan Georg
TARUTTIS Patentanwaltskanzlei
Aegidientorplatz 2b
30159 Hannover (DE)**

(54)     **Avian cell line and its use in production of protein**

(57)      The invention provides a process for generating an avian cell line of duck origin by a process comprising:
- cultivating embryonic avian cells in cell culture medium, preferably comprising fetal calf serum (FBS) for more than 40 passages while reducing the concentration of FBS to 1 - 2% vol/vol FBS,

- transferring passaged cells into cell culture medium having 0% FBS,
- resulting in the generation of a non-embryonic avian cell line suitable for non-adherent growth, i.e. for growth in suspended culture.

EP 2 660 316 A1

**Description**

The present invention relates to an avian cell line, suitable for protein production by heterologous expression, e.g. of soluble protein or of viral particles by cultivation of the cells, preferably in suspension in serum-free medium. Further, the invention relates to a process for producing the cell line and to the cell line produced by the process.

Due to the process for generating the cell line of the invention, the cell line has the advantage of being free from mammalian virus, e.g. free from viral nucleic acid sequences originating from virus having specificity for mammals.

**State of the art**

EP 1483369 B1, EP 1646715 B1 and EP 1985305 Al describe processes for generating avian cell lines using a specific combination of growth factors and cultivation on a mammalian feeder cell layer, followed by a change of medium involving the stepwise reduction of growth factors, serum components and feeder layer for adaptation of cells to cultivation conditions without feeder cells, and with a reduced content of growth factors and/or of serum. The cell lines carry embryonic stem cell markers, including surface markers SSEA-1, SSEA-3, and show cell renewal.

WO 2005/007840 Al describes the production of viral particles using an avian cell line generated by cultivating avian embryonic stem cells on an inactivated feeder cell layer in the presence of growth factors with subsequent stepwise withdrawal of feeder cells and of growth factors during subsequent passages. The final cell lines have telomerase and alkaline phosphatase activities.

Avian cells which have come into contact with mammalian cells have the disadvantage of possibly being contaminated with mammalian virus originating from the mammalian cells.

WO 2005/042728 Al describes a non-virally transfected cell line which was immortalized by transfecting a first gene disrupting the complex between retinoblastoma protein and E2F transcription factors and by transfecting a second gene affecting protein p53.

**Objects of the invention**

It is an object to provide an alternative avian cell line, especially a duck cell line, a method for generating the cell line, and a production process for protein, especially for soluble protein or for viral particles, using the cell line. It is a preferred object that the alternative cell line is suitable for suspended growth cultivation in a serum-free cell culture medium and is free from endogenous mammalian viral nucleic acid sequences, especially free from an integrated genome of a virus having specificity for mammalian cells.

Further, it is preferred that the alternative cell line has a high productivity for both heterologous soluble protein, e.g. for a protein having antigen specificity, especially for a natural or synthetic antibody, and for viral particles, e.g. for proteinaceous viral particles, optionally containing a synthetic nucleic acid construct or a viral genome, e.g. of an attenuated virus.

**General description of the invention**

The invention achieves the objects by the features of the claims, especially by a process for generating an avian cell line, preferably of duck origin, especially originating from Anas platyrhinchos or a domesticated breed thereof, by the following process:

- provide avian cells, preferably derived from an avian embryo, preferably from a duck embryo, aged 10 - 15 days, preferably of day 13,
- cultivate these avian cells in cell culture medium, preferably comprising fetal calf serum (FBS), e.g. at 8 - 12%, preferably at 12% vol/vol, e.g. modified MEM medium, e.g. in modified AdMEM medium with passaging of cells while reducing the concentration of FBS gradually preferably to about 1 - 4% vol/vol, e.g. to 2% vol/vol,
- for more than 40 passages, preferably for more than 50 passages, reducing the concentration of FBS to 1 - 2% vol/vol FBS, preferably to approximately 1%,
- preferably in static culture, e.g. in cell culture flasks under standard cultivation conditions, e.g. at 37°C in a 5% $CO_2$ atmosphere without agitation,
- transfer of passaged cells into cell culture medium having 0% FBS, especially 0% animal serum,
- resulting in the generation of a non-embryonic avian cell line suitable for non-adherent growth, i.e. for growth in suspended culture.
- Preferably, the non-embryonic avian cell lines suitable for non-adherent growth are adapted to growth in suspension by cultivating in ExCell EBxGro-I medium (available from SAFC Biosciences, Andover, UK) supplemented with glutamine according to the manufacturer's instructions, preferably for a number of subsequent passages, e.g. for at least 35 or at least 40 passages, e.g. for 140 passages, preferably at 37°C in a 5-7.5% $CO_2$ atmosphere.

Preferably, the avian embryo is a duck embryo, e.g. of Anas platyrhrinchos or of a domesticated breed originating therefrom.

A preferred example for a cell culture medium, especially suitable for cultivating the cells in suspended culture has a composition comprising or consisting of the following ingredients: MEM: L- arginine hydrochloride at 126 mg/L, L- cystine at 24 mg/L, L- glutamine at 292 mg/L, L- histidine hydrochloride- $H_2O$ at 42 mg/L, L- isoleucine at 52 mg/L, L- leucine at 52 mg/L, L- lysine hydrochloride at 73 mg/L, L- methionine at 15 mg/L, L- phenylalanine at 32 mg/L, L- threonine at 48 mg/L, L- tryptophan at 10 mg/L, L- tyrosine at 36 mg/L, L- valine at 46 mg/L, choline chloride at 1 mg/L, D- calcium pantothenate at 1 mg/L, folic acid at 1 mg/L, niacinamide at 1 mg/L, pyridoxal hydrochloride at 1 mg/L, riboflavin at 0.1 mg/L, thiamine hydrochloride at 1 mg/L, i- inositol at 2 mg/L, calcium chloride ($CaCl_2$- $2H_2O$) at 264 mg/L, magnesium sulfate ($MgSO_4$- $7H_2O$) at 200 mg/L, potassium chloride (KCl) at 400 mg/L, sodium bicarbonate ($NaHCO_3$) at 2200 mg/L, sodium chloride (NaCl) at 6800 mg/L, sodium phosphate monobasic ($NaH_2PO4$- $2H_2O$) at 158 mg/L, D- glucose (dextrose) at 1000 mg/L, phenol red at 10 mg/L.

AdMEM:

Glycine at 7.5 mg/L, L- alanine at 8.9 mg/L, L- arginine hydrochloride at 126, L- asparagine at 13.2 mg/L, L- aspartic acid at 13.3 mg/L, L- cystine 2HCl at 31 mg/L, L- glutamic acid at 14.7 mg/L, L- histidine hydrochloride- $H_2O$ at 42 mg/L, L- isoleucine at 52 mg/L, L- leucine at 52 mg/L, L- lysine hydrochloride at 72.5 mg/L, L- methionine at 15 mg/L, L- phenylalanine at 32 mg/L, L- proline at 11.5 mg/L, L- serine at 10.5 mg/L, L- threonine at 48 mg/L, L- tryptophan at 10 mg/L, L- tyrosine disodium salt dihydrate at 52 mg/L, L- valine at 46 mg/L, ascorbic acid phosphate at 2.5 mg/L, choline chloride at 1 mg/L, D- calcium pantothenate at 1 mg/L, folic acid at 1 mg/L, niacinamide at 1 mg/L, pyridoxine hydrochloride at 1 mg/L, riboflavin at 0.1 mg/L, thiamine hydrochloride at 1 mg/L, i- inositol at 2 mg/L, calcium chloride ($CaCl_2$) (anhyd.) at 200 mg/L, magnesium sulfate ($MgSO_4$) (anhyd.) at 97.67 mg/L, potassium chloride (KCl) at 400 mg/L, sodium bicarbonate ($NaHCO_3$) at 2200 mg/L, sodium chloride (NaCl) at 6800 mg/L, sodium phosphate dibasic ($Na_2HPO_4$- $H_2O$) at 140 mg/L, AlbuMAX® II at 400 mg/L, human transferrin (holo) at 7.5 mg/L, insulin recombinant full chain at 10 mg/L, ammonium metavanadate at 0.0003 mg/L, cupric sulfate at 0.00125 mg/L, manganous chloride at 0.00005 mg/L, sodium selenite at 0.005 mg/L, D- glucose (dextrose) at 1000 mg/L, ethanolamine at 1.9 mg/L, glutathione (reduced) at 1 mg/L, phenol red at 10 mg/L, sodium pyruvate at 110 mg/L.

A preferred example of a cell line according to the invention is deposited under the Budapest treaty under accession number DSM ACC3149, deposited on 12.December 2011 at the Deutsche Stammsammlung für Mikroorganismen und Zellkulturen (DSMZ), Inhoffenstr. 7, 38124 Braunschweig. Herein, this cell line is also termed sEFB1. This cell line is also the preferred cell line for the process for production of protein, e.g. of a soluble protein, e.g. a natural or synthetic antibody, or for a viral particle, preferably from a nucleic acid construct that encodes the protein within an expression cassette and is contained in the cell line. Surprisingly, it has been found that an avian cell line can be generated by this process, which cell line both has not been contacted with mammalian cells, e.g. has not been contacted with a feeder cell layer containing mammalian cells, and can be cultivated in suspension in a serum-free medium. It has been found that the cell lines generated by the process are genetically stable and differentiated, showing a fibroblast-like morphology and e.g. lack at least certain embryo-specific markers, e.g. preferably are free from the markers SSEA-1 (stage-specific embryonic antigen 1), SSEA-3 (stage-specific embryonic antigen 3), and EMA-1 (a primordial germ cell marker). The reduction or absence of embryonic markers shows that the cells of the invention are essentially differentiated. In contrast, cells previously described (e.g. EB66) exhibit embryonic markers and therefore retain at least some residual potential for unwanted differentiation and instability.

Further, the cell line of the invention is suitable for protein production at significant concentrations, e.g. by expression of a protein encoded by an expression cassette introduced into the cells, and the cell line is also suitable for production of viral particles at significant titers, including both virus having specificity for mammalian cells, especially for human cells, and virus having specificity for avian cells, especially for duck cells.

The process for generating a cell line of the invention shows that the limit to propagation, which can often be found to limit proliferation to approximately 35 to 40 passages can be overcome, even allowing a later reduction, e.g. following at least 35 passages, preferably more than 40 passages, while reducing the content of FBS to about 1 - 2%, followed by an adaptation of the cells to serum-free medium by further passaging in serum-free medium. The cell line of the invention can e.g. be propagated to at least 140 passages in serum-containing medium, preferably in serum-reduced medium, more preferred in serum-free medium without displaying a deterioration of growth characteristics or of protein production, including production of soluble and/or viral particles.

The cell lines of the invention combine the properties of being differentiated cells, i.e. with a reduced level of expressed embryonic cell specific marker, preferably without embryonic cell specific markers, especially without embryonic stem cell specific markers, but having the ability for growth, especially in suspended culture, e.g. for more than 50 passages, preferably for more than 70 passages or for more than 140 passages, preferably for unlimited propagation and unlimited proliferation under culture conditions, e.g. in serum-free medium, generally including cultivation in suspension. Preferably, the cell lines of the invention have a reduced level, e.g. of less than 10%, preferably of less than 5%, more preferably of less than 1% or of less than 0.1%, most preferably of less than 0.01 % of the level detected in embryonic cultivated cells of Anas platyrhynchos at passage 1, or are free from at least one, preferably from all of the following embryonic cell specific markers: SSEA-1, SSEA-3, and EMA-1. A reduced level of the embryonic cell specific markers is preferably determined by a specific assay, e.g. by an immunological assay for SSEA-1 and SSEA-3 (using anti-SSEA-1 and anti-SSEA -3 antibodies available from DSHB, Developmental Studies Hybridoma Bank, University of Iowa), and by an immunological. assay for EMA-1, respectively. Preferably, a cell line of the invention is free from the marker if in the specific assay the detected signal shows the absence of marker, e.g. if the detected specific signal is background level.

For the purposes of the invention, the growth or cultivation of cells in suspended culture or in suspension is the growth of single or agglomerated cell lines of the invention, preferably without attachment to an artificial surface, e.g. without attachment to a carrier.

The process for producing protein includes the cultivation of a cell line obtainable by the process for generating the cell lines, preferably in a serum-free cell culture medium, wherein the cells have been genetically manipulated to contain an expression cassette encoding the protein, and/or in the presence of a viral particle which propagates in the cells, optionally in the presence of an auxiliary factor allowing infection of the cells by the viral particle.

As an example for a sequence encoding the protein to be expressed by the cells, the expression cassette may contain a nucleic acid sequence encoding the protein to be produced, e.g. a viral particle and/or a viral coat protein or a protein having affinity to an antigen, like e.g. an antibody having a natural or synthetic amino acid sequence. Further examples for a protein are peptide hormones, cellular receptors, growth factors, cytokines or sections thereof. Generally, it is preferred that the expression cassette is introduced into the cells without additional bacterial nucleic acid sequence sections. For example, it is preferred that the expression cassette is contained in a nucleic acid construct comprising only eukaryotic sequence sections, e.g. comprising or consisting of the expression cassette, optionally a eukaryotic selection marker, nucleic acid sequence sections having homology to genomic nucleic acid sequence sections of the cells line for allowing homologous recombination, and/or fused remainders of recombinase recognition sites flanking the expression cassette, e.g. introduced by transient or permanent transfection.

As an example for viral particles, the cells can be contacted with attenuated virus, e.g. for producing a vaccine comprising the viral particles in a pharmaceutically acceptable formulation. Examples for viral particles are the modified Ankara virus strain of the vaccinia virus, attenuated strains of virus having specificity for humans and/or animals.

Generally, the process for producing protein, preferably soluble protein, comprises the steps of

- transfecting, e.g. permanently or transiently, the cell line with a nucleic acid construct containing an expression cassette containing a nucleic acid sequence encoding the protein to be produced, e.g. functionally linked to a constitutive or inducible promoter element,
- cultivating the cell line, preferably in serum-free medium, most preferably in suspended culture
- optionally inducing the promoter of the expression cassette by applying an inductive stimulus, e.g. an inductor substance,
- cultivating the genetically manipulated cells further,
- separating the protein expressed from the expression cassette from the cultivation medium and/or from the cells, optionally after separating cells from the culture medium.

For producing viral particles using the cell line, the process preferably comprises the steps of

- cultivating the cell line, preferably in suspended culture, most preferably in serum-free medium,
- adding the viral particle,
- optionally adding an auxiliary agent, e.g. a protease in combination with hemagglutinin,
- optionally separating the cells from the medium,
- isolating viral particles from the culture medium,
- and formulating a pharmaceutically acceptable composition comprising the viral particles, optionally including the step of inactivating the viral particle.

The viral particle can be an attenuated virus, a wild-type virus, or comprise a synthetic nucleic acid sequence in viral coat protein.

**Detailed description of the invention**

The invention is now described in greater detail with reference to the figures, wherein

- Figure 1 shows a growth-curve of the cell line when cultivated in suspension,
- Figure 2 shows a growth-curve of the cell line in suspended culture in serum-free medium,
- Figure 3 shows a growth-curve of the cell line in suspension with infection with an infective viral particle used for virus production,
- Figure 4 shows the specific productivity for a viral particle on the example of a virus in suspended growth of cells,
- Figure 5 shows data of the production of a secreted heterologous protein from a comparative cell line,
- Figure 6 shows data of the production of a secreted heterologous protein in a cell line of the invention,
- Figure 7 shows data of the relative expression of a heterologous gene after transient transfection of the cell line in comparison to a comparative cell line from the same expression cassette,
- Figure 8 schematically shows nucleic acid constructs containing a first expression cassette for a first heterologous protein and a second expression cassette using an IRES sequence for a second heterologous protein,
- Figures 9 and 10 show data of the production of the first and second heterologous proteins, respectively, under the control of different promoters and IRES sequences of the first and second expression cassettes, respectively,

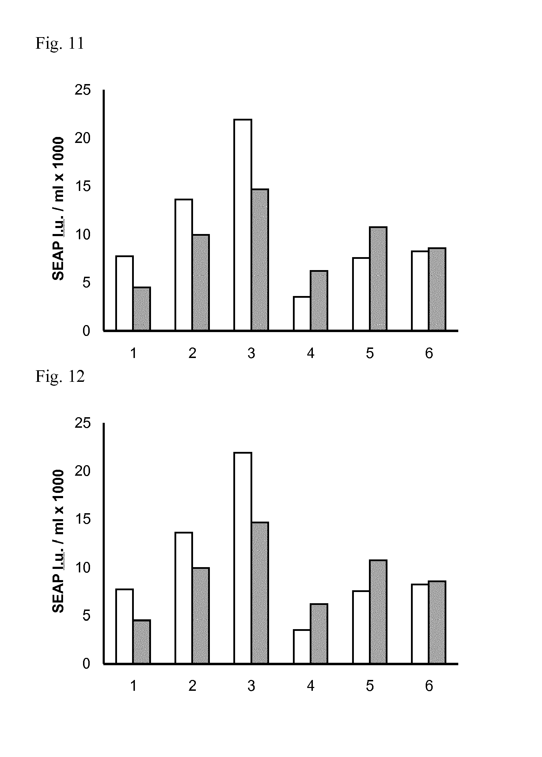

- Figures 11 and 12 show data of the expression of heterologous proteins from nucleic acid constructs under the control of a promoter or an IRES element, respectively, after transient transfection at days 1 and 2 following transfection, respectively.

Example 1: Generation of an avian cell line

A cell line of the invention was generated from avian cells obtained from embryonic tissue of an Anas platyrhinchos embryo at day 13, cultivated in MEM, supplemented with 10% FBS for 45 - 50 passages, each passage comprising growth under cell culture conditions in static cell culture flasks to 70 - 100% confluence. It was observed that at passage 40, cell proliferation was significantly decreased. Passaging was continued until passage 144. In further passaging, e.g. in the subsequent 8 passages, the concentration of serum was reduced, in one step or, as preferred in steps of 0.5 to 1%, to a total of 2% FBS in cell culture medium, preferably in AdMEM, with subsequent further passaging in medium with a content of 1% FBS.

Following at least 5 passages in 1% FBS medium, passaging was continued into serum-free medium, preferably having a composition of serum-free medium EBx GRO-I without glutamine, suitable for embryonic stem cells as available from SAFC Biosciences, Andover (UK) under catalogue No. 14530C, preferably with glutamine added according to the manufacturer's instructions. During cultivation in serum-free medium, it was observed that cells started to grow in free suspension. The cells could be transferred to a bioreactor for suspended growth, e.g. with agitation of the serum-free medium, e.g. by orbital shaking. One cell line generated by this process was deposited under accession number DSM ACC3149.

The cell line growing in serum-free medium in suspension could be used for expression of heterologous soluble protein or, using infection by a viral particle, for production of attenuated virus.

During the subsequent passaging steps, the growth was monitored by measuring the doubling time. Susceptibility to infection was measured by determination of the infectious titer of an aliquot of the adherent cells using a virus, e.g. Influenza A Puerto Rico 8/34/ (Mount Sinai strain), using the plaque assay on an MDCK cell layer. Optionally, the hemagglutination reaction can be used, which is also applicable for non-infectious virus particles. Neuraminidase activity was measured in cells growing in suspension by the NA-STAR assay (available from Applied Biosystems, USA), and the heterologous protein expression was measured using a reporter gene assay by transfecting the cells with a nucleic acid construct containing an expression cassette encoding a reporter gene, e.g. G-Luc (Gaussia Luciferase), C-Luc (Cypridina Luciferase) as a reporter gene by measuring luciferase activities (e.g. using the GLuc or C-Luc reporter gene assay available from NEN BioLabs).

The doubling or generation times were determined to 22h at passage 7 and to 28.8h at passage 30. The population doubling time at passage 146 was determined to 20.1h in AdMEM, 1% FBS, conditioned by adding the same volume of 1d culture supernatant obtained from a confluent culture of EFB1 (duck fibroblast) cells in the same medium, and to 28.7h in AdMEM, 1% FBS without this conditioning.

In the plaque assay, an infectious titer of 11-17 pfu/cell and day was determined at passages 58-66 for the cell line cultivated in 1% FBS AdMEM medium.

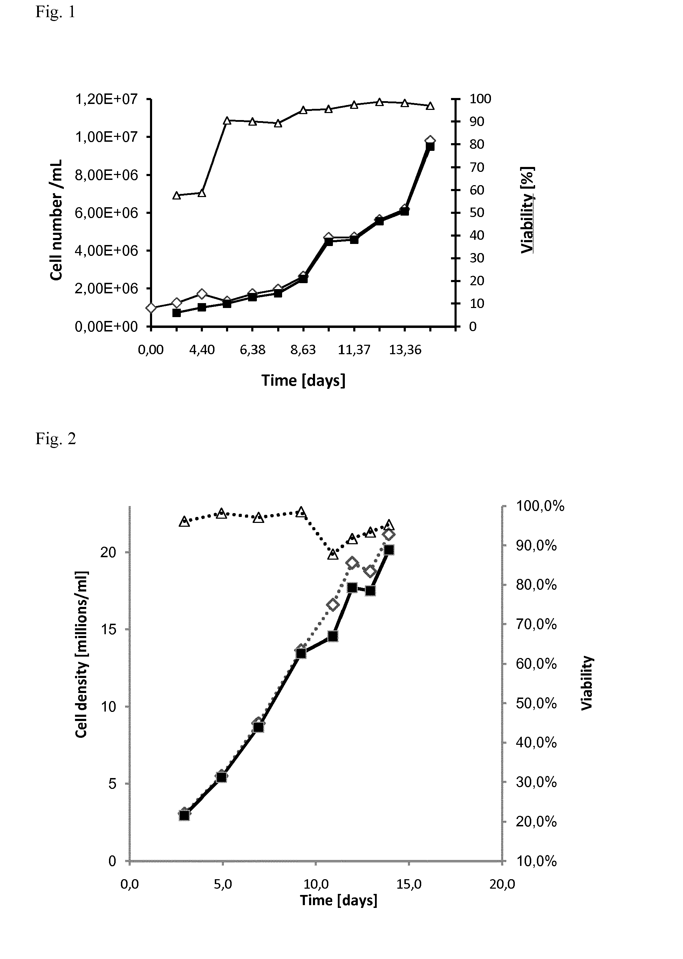

Figure 1 shows the growth of sEFB1 cells of the invention in 50 mL serum-free EX-CELL EBx GRO-I medium (obtained as cat. No. 14530C, lot 10B365 , SAFC Biosciences) with orbital shaking (25 mm orbit, 110 rpm) in 125 mL shake flasks. The starting cell concentration was $10^6$ cells/mL, medium was exchanged every 2 days. The total

cell number

($\diamond$), number of vital cells (■) and viability in % ($\Delta$, viable cells) are given, reaching maximal values at approximately day 14-15, at a maximal cell density of $1\times10^7$ cells/mL. The generation time was determined to 60-72h for the exponential growth phase.

Figure 2 shows the growth of the sEFB1 cells of the invention in 50 mL serum-free EX-CELL EBx PRO-I medium (obtained as cat. No. 14531C, lot RNBB6216 from SAFC Biosciences), which can be used for protein or viral particle production, with orbital shaking (25 mm orbit, 110 rpm) in 125 mL shake flasks. In the beginning, medium was exchanged every 2 days, and from day 9, medium was exchanged every day. The total cell number ($\diamond$), number of vital cells (■) and viability in % ($\Delta$) are given for the suspended culture, indicating a maximal cell density of $2\times10^7$ cells/mL at a generation time of approx. 69h in the exponential growth phase. This culture was terminated at day 13 after the last sampling. In comparison to the culture shown in Fig. 1, a two-fold higher maximum cell density was reached.

As preferred, cells were passaged for at least 40 passages in ExCell EBxGro-I medium supplemented to a final concentration of 2.5 mM glutamine.

Example 2: Production of Influenza A virus

Using a cell line generated according to Example 1, human influenza virus A/Puerto Rico/8/1934 H1N1 (Influenza A PR8) was produced as an example for a viral particle. Briefly, cells were grown in static culture in 1% FBS MEM medium to confluence and infected with a multiplicity of infection (MOI) of 0.2. Following further incubation for 24h under cell culture conditions. The titer of viral particles was determined from culture supernatant by the plaque assay on MDCK cells.

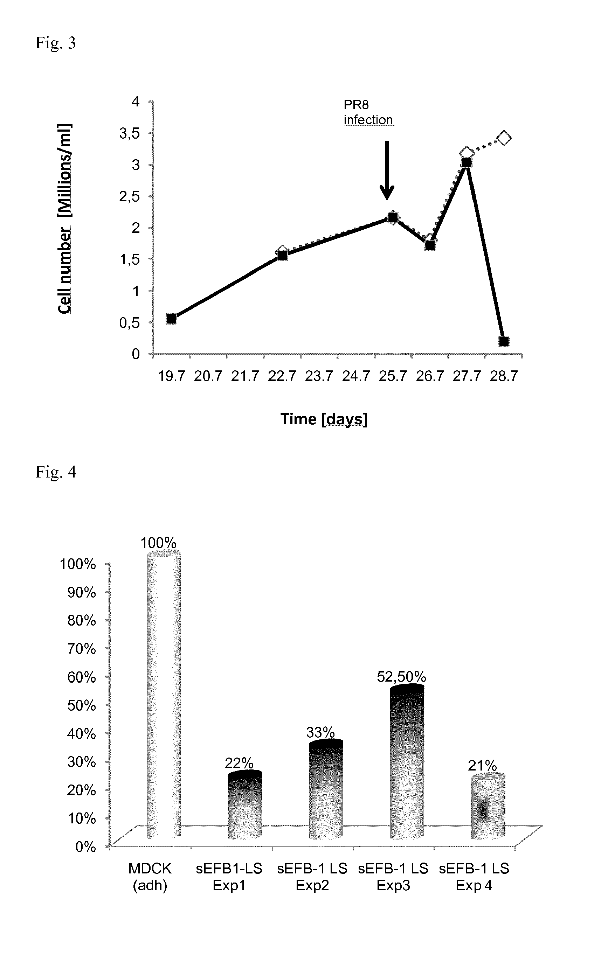

Figure 3 shows the cell growth of SEFB1 of the invention infected with Influenza A virus PR8 strain at an MOI of 0.01 at day 6 of cultivation in suspension , as indicated by the arrow for addition of the infectious viral particles. Cells were cultivated in 10 mL growth medium (EBx GroI) in a bioreactor 50 (TPP, 50ml on an orbital shaker (1 cm orbit, 220 rpm). The lytic effect of the virus is evident.

Figure 4 shows the relative productivity of the cells of the invention for Influenza A PR8 in the neuraminidase assay (NA_STAR) with luminescence detection in comparison to virus production in adherently growing (adh) MDCK cells (100%). Neuraminidase activity (NA) was determined using a luminometric assay (NASTAR, Applied Biosystems). NA reflects viral titers in the culture supernatant. Parallel experiments (Exp) 1 to 4 for the cell line of the invention show that 21-53% of the productivity in MDCK cells was reached in these initial experiments. In experiments 1-3, EX-CELL EBx GRO-I medium was used, and EX-CELL EBx PRO-I medium was used in experiment 4.

Example 3: Production of soluble secretory protein in suspension culture

For expression of a soluble protein, an expression cassette encoding secretory Cypridina luciferase (C-Luc) under the control of the SV40 promoter was transiently introduced into cells generated according to Example 1 and into HEK 293s suspension cells for comparison. The expression cassette was contained in a bacterial shuttle plasmid. Cells were transfected by contacting cells with the DNA containing the expression cassette in vector pSV-CLuc in the presence of polyethylene imine (PEI).

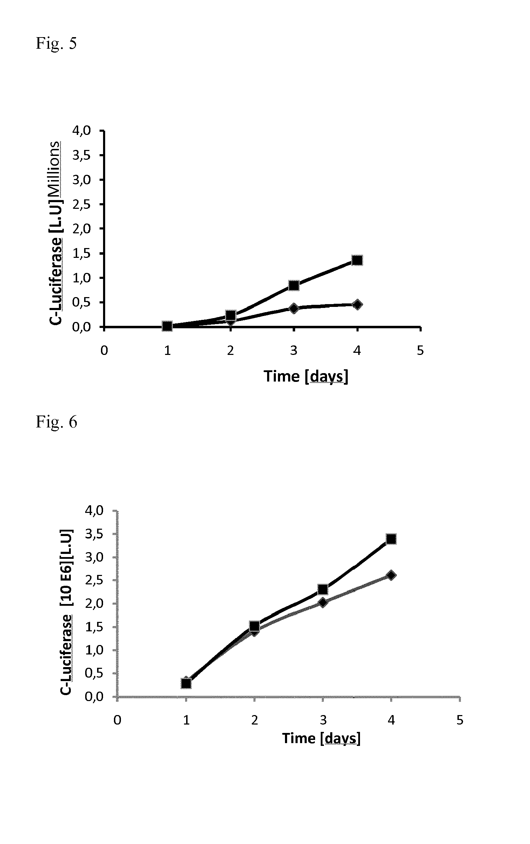

For cultures of suspended cells, Figure 5 shows the activity of secreted C-Luc in cell-free medium of the comparative 293s cell culture under the control of the SV40e (SV40 early) promoter. Cells were cultivated in 10 mL growth medium (SFM II) in a bioreactor 50 (TPP) on an orbital shaker (1 cm orbit, 220 rpm). Cells were transfected with the plasmid containing an expression cassette comprising SV40e C-Luc using a 3:1 ratio of PEI:DNA. Cells were cultivated for the indicated duration, Cypridina luciferase (C-Luc) production was determined daily from supernatant after 10-fold dilution in a luminometric assay. Light units are indicated as l.u.

Figure 6 shows the activity of secreted C-Luc in cell-free medium of a cell line of the invention, sEFB1. Cells were cultivated in 10 mL growth medium (EBx Pro-I) in a bioreactor (BioReactor 50, TPP) with orbital shaking (1 cm orbit, 220 rpm). Cells were transfected with the plasmid containing an expression cassette comprising SV40e C-Luc using a 3:1 ratio of PEI:DNA. Cells were cultivated for the indicated duration, Cypridina luciferase (C-Luc) production was determined daily from supernatant after 10-fold dilution in a luminometric assay. $\diamond$ and ■ indicate results from individual experiments using pSV-CLuc. Measurement results show that soluble secretory protein was produced to a 3-fold higher concentration in the cell line of the invention in comparison to suspended HEK 293s cells.

Example 4: Production of soluble non-secretory protein in suspension culture

For expression of a non-secretory soluble protein, an expression cassette encoding firefly luciferase (F-Luc) under the control of the human cytomegalovirus promoter (hCMV) was constructed on a minicircle, i.e. on a plasmid devoid of sections of bacterial origin and transiently transfected into comparative HEK 293s cells and in cells generated according to Example 1, respectively, using PEI for transfection. Briefly, the minicircle did not contain nucleic acid sections used for replication in bacteria and was produced by internal recombination using Flp recombinase on the two Flp-specific FRT recognition sites flanking the expression cassette. As a result of the recombination, the minicircle essentially consisted of the expression cassette containing the coding sequence for the protein (F-Luc) between an upstream promoter element and a downstream polyadenylation site and the joined remainders of the FRT recombinase recognition sites.

Luciferase activities were measured for 4 days from cells isolated from the culture, and cell number was determined for calculation of relative activities. Productivity values are normalized to cell number and daily production. It was found that non-secretory protein can be produced in the cells of the invention in suspended culture.

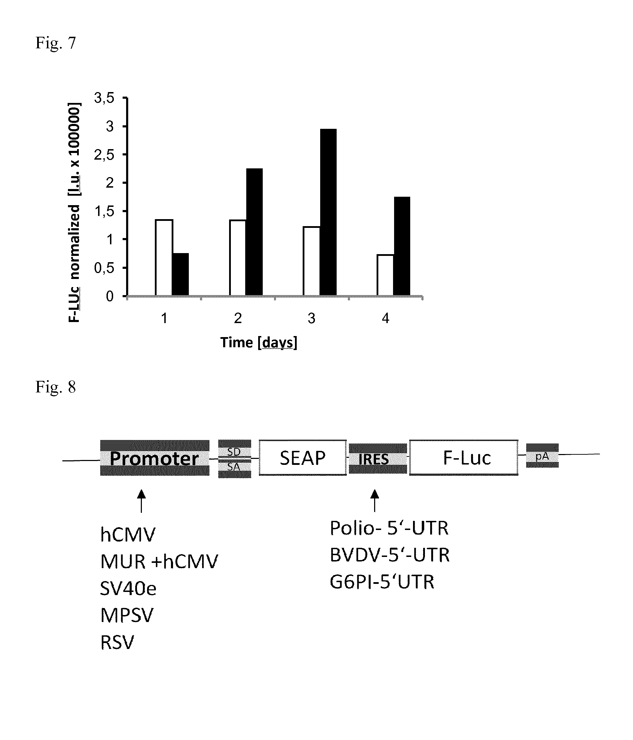

Further, it was found that minicircles perform equally well in 293s cells and in sEFB1 cells of the invention. As shown in Fig. 7, in sEFB1 cells, the luciferase expression was approx. threefold higher from minicircles (black bar) compared to the original plasmids (white bar) containing sequence sections of bacterial origin (ori, selection marker ) . The higher expression from minicircle compared to plasmids was also found in HEK 293s cells.

Example 5: Production of two soluble proteins from coupled expression cassettes in suspension culture

In order to elucidate the effect of promoter strength, the efficiency of elements allowing internal intiation of translation and of the arrangement of sequences encoding the protein, two expression cassettes were coupled, a first expression cassette containing a promoter upstream a first protein coding sequence, and in 3' an adjacent second expression cassette containing an IRES element as a promoter element upstream a second protein coding sequence, with a polyadenylation (pA) element downstream of the second protein coding sequence. The first protein coding sequence was secreted alkaline phosphatase (SEAP), which due to being secretory was analysed from the cell-free culture medium. A splice donor site and a splice acceptor site (SD, SA) were arranged between the promoter and the first coding sequence. The second coding sequence encoded the non-secretory F-Luc. The results show that the cells of the invention can be used in a process for protein production from mono-cistronic and from bi-cistronic expression vectors, e.g. shuttle plasmids or minicircles.

The genetic transcriptional control elements (i.e. promoters) were chosen from the human cytomegalovirus immediate early (IE) promoter/ enhancer (hCMV), the modulator and unique region (MUR) upstream of the human cytomegalovirus IE promoter combined with the human cytomegalovirus immediate early (IE) promoter/ enhancer (hCMV) (MUR CMV),
the Simian virus 40 early promoter (SV40e),
the long terminal repeat region (LTR) of the myeloproliferative sarcoma virus harbouring the retroviral promoter (MPSV),
the Rous sarcoma virus promoter (RSV) .
The translational control elements (internal ribosome entry sites, IRES) were chosen from the poliovirus 5'- untranslated region (Polio- 5'- UTR),
the bovine viral diarrhea virus 5'- UTR (BVDV- 5'- UTR), and
the glucose- 6- phosphate isomerase 5'- UTR (G6PI- 5'- UTR) .

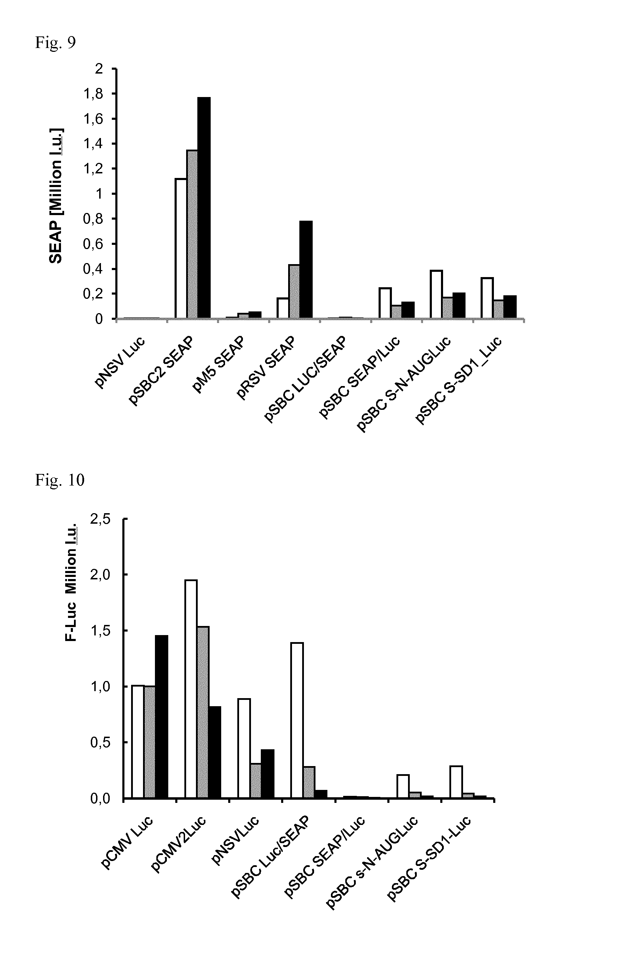

The coding sequence was SEAP, the secreted alkaline phosphatase gene, and F-Luc, the firefly luciferase gene, respectively. The splice donor (SD) and splice acceptor (SA) regions were from SV40e, the polyadenylation site (pA) originates from the SV40 early pA. Figure 8 schematically shows the arrangement of the genetic elements. Figures 9 and 10 show data for protein expression by way of activities of the proteins expressed. In each set of columns, the left column indicates the activity 1 day after transfection, the centre column indicates the activity after 2 days, and the right column indicates the activity after 3 days.

Cells were transfected with plasmids containing one of the monocistronic and bicistronic expression cassettes. The SV40 early promoter was contained in pNSV Luc and pSBC cassettes (Dirks et al. Gene128, 247-249 (1993), the promoter from MPSV LTR (myeloproliferative sarcoma virus long terminal repeat) in pM5 SEAP (pM5 is a vector containing the LTR from MPSV), the promoter from RSV-LTR (Rous sarcoma virus long terminal repeat) in pRSV SEAP, the IRES from poliovirus type I between coding sequences SEAP and F-Luc (pSBC SEAP/Luc), the IRES BVDV-5'-UTR was contained between coding sequences SEAP and F-Luc.

The results show that generally the cell lines of the invention are suitable for simultaneous production of at least two proteins, at least one of which can be a secretory and/or a non-secretory protein, which can be encoded by adjacent expression cassettes on one common nucleic acid construct, e.g. on an at least bicistronic nucleic acid construct. Further, the results show that the expression level of each protein is dependent on the promoter element functionally coupled to the coding sequence, and that accordingly, the relative expression of different proteins produced in a cell line can be controlled by the promoter element functionally coupled to the coding sequence.

It was found that from the promoter elements tested, the promoter strength in the cell line, i.e. the relative level of expression from the coding sequence functionally coupled to the region having promoter activity, is

$$MUR\ CMV > hCMV \sim SV40e > RSV\ LTR \gg MPSV\ LTR$$

and for the elements suitable for internal initiation of translation of mRNA, IRES, the strength of initiation of translation is BVDV >> G6PI - poliovirus.

## Claims

1. Process for generating an avian cell line, comprising the steps of

   - providing avian cells, preferably derived from an avian embryo,
   - cultivating these avian cells in cell culture medium comprising fetal calf serum (FBS), with passaging of cells while reducing the concentration of FBS gradually
   - for more than 40 passages, reducing the concentration of FBS to 1 - 2% vol/vol,
   - in static culture at 37°C in a 5% $CO_2$ atmosphere,
   - transferring of passaged cells into cell culture medium having 0% FBS,
   - resulting in the generation of a non-embryonic avian cell line suitable for non-adherent growth.

2. Process according to claim 1, **characterized in that** the avian embryo is a duck embryo, aged 10 - 15 days.

3. Process according to one of the preceding claims, **characterized in that** the cell line is non-embryonic due to absence of marker SSEA-1.

4. Process according to one of the preceding claims, **characterized in that** the cell line is DSM ACC3149.

5. Process according to one of the preceding claims, **characterized in that** the cell line is susceptible for infection by virus having specificity for mammals and upon transfection with a nucleic acid construct comprising an expression cassette encoding a protein is capable of expressing the encoded protein.

6. Avian cell line for use in the production of protein, **characterized in that** the cell line is obtainable by a process according to one of the preceding claims.

7. Process for producing protein comprising the cultivation of an avian cell line, **characterized in that** the avian cell line is one according to claim 6.

8. Process according to claim 7, **characterized in that** the cell line is transfected with a nucleic acid construct containing at least one expression cassette containing a nucleic acid sequence encoding the protein.

9. Process according to one of claims 7 and 8, **characterized in that** the protein is a natural or a synthetic antibody having specificity for an antigen.

10. Process according to claim 7, **characterized in that** the protein is a viral particle and the cell line is infected with an infectious viral particle.

11. Process according to claim 10, **characterized in that** the viral particle is an attenuated virus and the process after cultivating the cell line in the presence of the viral particle comprising the step of separating the viral particles from the cell line.

12. Process according to claim 10, **characterized in that** the viral particle is a wild-type virus which is infectious for human cells or for non-human animal cells, the process after cultivating the cell line in the presence of the viral particle comprising the step of separating the viral particles from the cell line and, previously or subsequently, inactivating the viral particles.

Fig. 1

Fig. 2

Fig. 3

Fig. 4

Fig. 5

Fig. 6

Fig. 7

Fig. 8

hCMV
MUR +hCMV
SV40e
MPSV
RSV

Polio- 5'-UTR
BVDV-5'-UTR
G6PI-5'UTR

Fig. 9

Fig. 10

Fig. 11

Fig. 12

Europäisches
Patentamt

European
Patent Office

Office européen
des brevets

# EUROPEAN SEARCH REPORT

Application Number

EP 12 16 6489

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 985 305 A1 (VIVALIS [FR]) 29 October 2008 (2008-10-29) * e.g. section 2.2 starting on page 28; example 7-9; the whole document * | 1-12 | INV. C12N5/0735 C12N7/00 |
| X | WO 2005/007840 A1 (VIVALIS [FR]; GUEHENNEUX FABIENNE [FR]; PAIN BERTRAND [FR]) 27 January 2005 (2005-01-27) * e.g. example 1, 5, 9, 12-14; page 24, line 25-26; the whole document * | 1-12 | |
| X | US 2011/294209 A1 (PAIN BERTRAND [FR] ET AL) 1 December 2011 (2011-12-01) * e.g. example 1,2,5,9,13; claim 1-3; the whole document * | 1-12 | |
| X | PHILIPPE-ALEXANDRE GILBERT ET AL: "Current Status for High Titre Poxvirus Stock Preparation in CEF Under Serum-Free Medium Conditions: Implication for Vaccine Development", CYTOTECHNOLOGY, KLUWER ACADEMIC PUBLISHERS, DO, vol. 48, no. 1-3, 1 June 2005 (2005-06-01), pages 79-88, XP019236893, ISSN: 1573-0778, DOI: 10.1007/S10616-005-3795-Y * e.g. abstract; the whole document * | 6-12 | TECHNICAL FIELDS SEARCHED (IPC) C12N |

-/--

The present search report has been drawn up for all claims

| Place of search | Date of completion of the search | Examiner |
|---|---|---|
| The Hague | 25 September 2012 | Gruber, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**Europäisches Patentamt**

**European Patent Office**

**Office européen des brevets**

# EUROPEAN SEARCH REPORT

Application Number

EP 12 16 6489

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VOLLMAR J ET AL: "Safety and immunogenicity of IMVAMUNE, a promising candidate as a third generation smallpox vaccine", VACCINE, ELSEVIER LTD, GB, vol. 24, no. 12, 15 March 2006 (2006-03-15), pages 2065-2070, XP028010537, ISSN: 0264-410X, DOI: 10.1016/J.VACCINE.2005.11.022 [retrieved on 2006-03-15] * e.g. page 2066, left-hand column, paragraph 2; the whole document * | 6-12 | |
| X | MALLANNA S K ET AL: "Inhibition of Anatid Herpes Virus-1 replication by small interfering RNAs in cell culture system", VIRUS RESEARCH, AMSTERDAM, NL, vol. 115, no. 2, 1 February 2006 (2006-02-01), pages 192-197, XP024957000, ISSN: 0168-1702, DOI: 10.1016/J.VIRUSRES.2005.08.012 [retrieved on 2006-02-01] * e.g. abstract; page 193, section 2.1; page 194, section 2.4; the whole document * | 6-12 | TECHNICAL FIELDS SEARCHED (IPC) |

The present search report has been drawn up for all claims

| Place of search | Date of completion of the search | Examiner |
|---|---|---|
| The Hague | 25 September 2012 | Gruber, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 12 16 6489

This annex lists the patent family members relating to the patent documents cited in  the above-mentioned European search report.
The members are as contained in the European Patent Office EDP file on
The European Patent Office is in no way liable for these particulars which are merely given for the purpose of information.

25-09-2012

| Patent document cited in search report | | | Publication date | Patent family member(s) | | | Publication date |
|---|---|---|---|---|---|---|---|
| EP 1985305 | A1 | | 29-10-2008 | AU | 2008240708 | A1 | 30-10-2008 |
| | | | | CA | 2684845 | A1 | 30-10-2008 |
| | | | | CN | 101668539 | A | 10-03-2010 |
| | | | | EA | 200970996 | A1 | 30-04-2010 |
| | | | | EP | 1985305 | A1 | 29-10-2008 |
| | | | | EP | 2150275 | A1 | 10-02-2010 |
| | | | | JP | 2010524482 | A | 22-07-2010 |
| | | | | KR | 20100017340 | A | 16-02-2010 |
| | | | | NZ | 581321 | A | 25-05-2012 |
| | | | | US | 2010062489 | A1 | 11-03-2010 |
| | | | | WO | 2008129058 | A1 | 30-10-2008 |
| | | | | ZA | 200908152 | A | 28-04-2010 |
| WO 2005007840 | A1 | | 27-01-2005 | AT | 467678 | T | 15-05-2010 |
| | | | | AU | 2004257939 | A1 | 27-01-2005 |
| | | | | CA | 2531565 | A1 | 27-01-2005 |
| | | | | DK | 1646715 | T3 | 16-08-2010 |
| | | | | EP | 1646715 | A1 | 19-04-2006 |
| | | | | EP | 2143788 | A1 | 13-01-2010 |
| | | | | ES | 2345820 | T3 | 04-10-2010 |
| | | | | HK | 1091229 | A1 | 05-11-2010 |
| | | | | JP | 4787156 | B2 | 05-10-2011 |
| | | | | JP | 2006527995 | A | 14-12-2006 |
| | | | | JP | 2011224009 | A | 10-11-2011 |
| | | | | KR | 20060057577 | A | 26-05-2006 |
| | | | | PT | 1646715 | E | 09-08-2010 |
| | | | | SI | 1646715 | T1 | 30-09-2010 |
| | | | | US | 2006233834 | A1 | 19-10-2006 |
| | | | | US | 2009239286 | A1 | 24-09-2009 |
| | | | | US | 2010111999 | A1 | 06-05-2010 |
| | | | | WO | 2005007840 | A1 | 27-01-2005 |
| US 2011294209 | A1 | | 01-12-2011 | AT | 408004 | T | 15-09-2008 |
| | | | | AU | 2003227820 | A1 | 22-09-2003 |
| | | | | CA | 2478125 | A1 | 18-09-2003 |
| | | | | CN | 1649999 | A | 03-08-2005 |
| | | | | CN | 101676388 | A | 24-03-2010 |
| | | | | DK | 1483369 | T3 | 01-12-2008 |
| | | | | EP | 1483369 | A1 | 08-12-2004 |
| | | | | EP | 1992687 | A2 | 19-11-2008 |
| | | | | ES | 2312775 | T3 | 01-03-2009 |
| | | | | FR | 2836924 | A1 | 12-09-2003 |
| | | | | JP | 2005525803 | A | 02-09-2005 |
| | | | | JP | 2012110344 | A | 14-06-2012 |
| | | | | PT | 1483369 | E | 17-12-2008 |
| | | | | SI | 1483369 | T1 | 31-12-2008 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.**

EP 12 16 6489

This annex lists the patent family members relating to the patent documents cited in the above-mentioned European search report.
The members are as contained in the European Patent Office EDP file on
The European Patent Office is in no way liable for these particulars which are merely given for the purpose of information.

25-09-2012

| Patent document cited in search report | Publication date | Patent family member(s) | | Publication date |
|---|---|---|---|---|
| | | US 2004058441 A1 | | 25-03-2004 |
| | | US 2009239297 A1 | | 24-09-2009 |
| | | US 2010221825 A1 | | 02-09-2010 |
| | | US 2011294209 A1 | | 01-12-2011 |
| | | WO 03076601 A1 | | 18-09-2003 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1483369 B1
- EP 1646715 B1
- EP 1985305 A
- WO 2005007840 A1
- WO 2005042728 A1

**Non-patent literature cited in the description**

- **DIRKS et al.** *Gene,* 1993, vol. 128, 247-249